# EUROPEAN PATENT APPLICATION

(11) **EP 2 732 788 A1**
(43) Date of publication of application: **21.05.2014**
(21) Application number: 12193198.4
(22) Date of filing: 19.11.2012
(51) Int. Cl.: A61B 19/00

(54) **A system for enabling precision placement of an implant in a patient undergoing surgery**

(71) Applicant: Metronor AS, 1394 Nesbru (NO)
(72) Inventor: Rotvold, Oyvind, 1395 Hvalstad (NO)
(74) Representative: Lang, Johannes

(57) **Abstract**

A system providing a set of targets 6 following the patient 4, an independent set of targets 6 following the implant 1, as well as a measurement camera 3 viewing these targets 6 where each set of targets 6 as well as the camera 3 may move or be moved independently relative to each other during measurement.

## Description

With steadily higher average life span for humans, there is an increasing need for procedures to replace parts of the human body such as e.g. hips and knees that may wear out prematurely. Artificial replacements or implants are positioned to restore the original body function, and the precision with which these implants are placed is of critical importance.

In Sweden alone, more than 10,000 such operations are carried out annually. An implant life of over 20 years is possible if a good fit is achieved, but it is estimated that about 15-20% of these operations are unsuccessful and cause the implant to be have to be replaced within 5 years due to failure to achieve a good fit.

For the patients, this problem means severely reduced quality of life, and it also places heavy financial burdens on the health services or insurance providers and occupies surgical resources that are in high demand.

As life expectancy increases, it also becomes increasingly important to ensure that the initial implant achieves a good fit in order to last long enough to avoid expensive and painful replacement surgery when the patient is of advanced age and will heal very slowly, or when a replacement surgery will be declined on cost grounds by the health services or insurance providers.

Improvements in imaging and surgical planning tools enable surgeons to determine the ideal implant size and shape, as well as the ideal position of the implant, prior to any surgery taking place. If the implant could also be placed as planned, surgery would be faster and involve less risk, healing would be accelerated and the end-result and recuperation time for the patient would improve.

An efficient system must meet a number of criteria, among the most important are:
- Ease of use in the surgery setting, including not interfering with the surgical team's movement and line-of-sight
- Ability to determine the position and orientation of multiple moving objects - such as body parts, implants and instruments - simultaneously
- Reasonable acquisition cost, low operating cost.

Systems for measuring the position and orientation in space of a pattern of targets, said pattern comprising at least 3 targets in known positions relative to each other are described for example in EP 607 303 B1.

It follows that the position and orientation of any object stiffly connected with the pattern of targets can therefore also be measured. Such an object might be a measurement probe, or it could be a part of an assembly one wishes to position into a specific placement - such as an implant - or an object one wishes to track through time and space - such as a bone structure undergoing surgery.

It is further known e.g. from EP 607 303 B1, that if the relative positions of the individual targets of a pattern of at least three targets is known - and certain constraints are placed on the target pattern - then the position and orientation in three-dimensional space of this pattern of targets relative to a camera can be determined from the two-dimensional picture of the targets taken by the camera.

If a picture is taken that contains two or more non-identical known patterns of targets, it therefore follows that it is possible to determine the spatial position and orientation of each of these patterns of targets relative to the camera. This enables a system derived from the teachings of EP 607 303 B1 to measure the position and orientation of several objects - for example, the femur and the implant - at the same time.

If the known patterns of targets are identical, their spatial position and orientation can still be determined provided (1) an initial position for each is known, and (2) subsequent movement between pictures is small enough to track each pattern through time, and (3) at no point in time are the two patterns overlapping or closer to overlapping than the movement since the previous picture.

Alternative systems with similar capabilities are presented e.g. in EP 1712 193 A1 and in DE 196 49 399 A1. Both of these systems are also able to determine the position and orientation of multiple objects, and like EP 607 303 B1, both require a stationary camera.

All measurements made with a system such as those described in EP 607 303 B1, EP 1712 193 A1 and DE 196 49 399 A1 are made in a reference frame tied to the camera, typically through an initial process of using a measurement probe and touching a number of reference features. This is stated explicitly in DE 196 49 399 A1, and is implicit in the description of EP 1712 193 A1 as one would otherwise need to establish some other reference frame to relate the measurements to.

Additionally, the IR sensors taught in EP 1712 193 A1 and DE 196 49 399 A1 are not capturing instantaneous scenes, but are - admittedly quickly - measuring point by point and cannot measure two or more points simultaneously as this would return the average of the two. This reduces accuracy slightly, as the different targets are captured at slightly different points in time. This is not critical, as the movements involved are relatively small. It does, however, require that the camera is stationary during measurement, as the measurement accuracy would otherwise be ruined due to the long distance between the sensor and the targets. In a camera-fixed coordinate system, a small angular movement of the camera will cause a large shift in the position of a target, and targets observed at even slightly different times will have large relative deviations - worse the further from the camera they are.

Therefore, it is essential for these known systems that the camera is held stationary, as the relationship with the references is otherwise lost.

Such systems are therefore ill suited for the practical implant placement task. During this type of surgery involving large forces and extensive mechanical forming of bone structure, it is impractical to keep the patient still, and also impractical to hold the camera still as it would either lose line-of-sight to the wound during surgery, or get in the way of the medical staff.

The object of the present invention is to provide a system that provides to surgeons precise spatial measurement data needed for precision placement of an implant in a patient undergoing surgery.

This object is achieved according to the invention by a system as defined in claim 1. Advantageous embodiments of the inventive system are defined in dependent claims 2 to 8.

The inventive system permits efficient and precise measurement of two or more moving or movable objects relative to each other, particularly of an implant relative to the element in a patient to which the implant is to be fixed. The present invention provides a set of targets following the patient, an independent set of targets following the implant, as well as a measurement camera viewing these targets where each set of targets as well as the camera may move or be moved independently relative to each other during measurement, thus providing the necessary flexibility to be a practical solution to the problem.

The invention is based on the principle that knowing the position and orientation of the two or more patterns of targets at given simultaneous instances in time, it is possible to calculate the relative position and orientation of the two or more targets to each other at the same instances, as well as the observation that this will hold true also if the camera is moved relative to the two patterns of targets, thus enabling a measurement system where the camera can be moved independently from any one or all of the patterns of targets and still provide measurements of their relative position and orientation to each other.

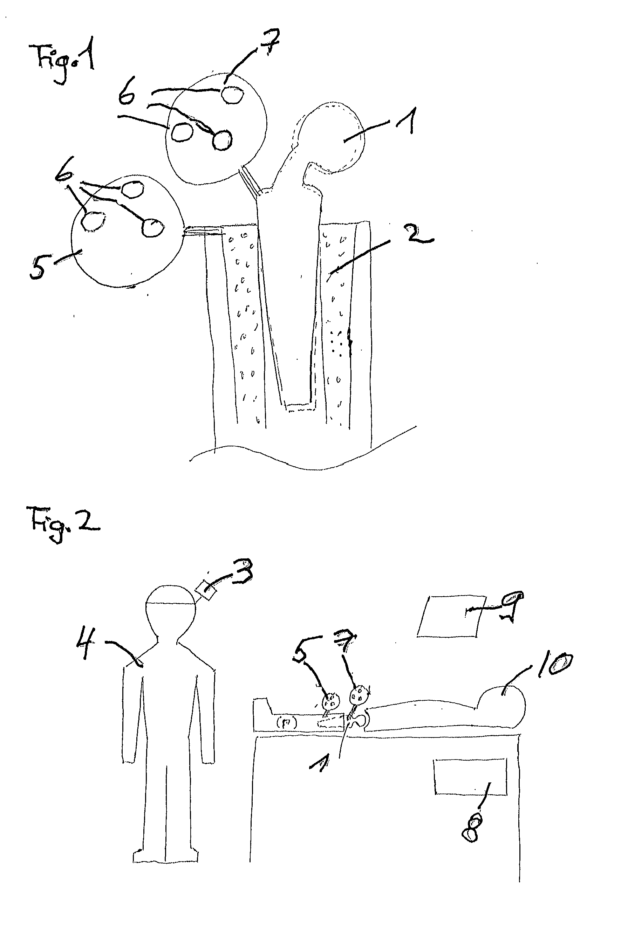

Further features, advantages and application possibilities of the invention can be taken from the following description of preferred embodiments together with the drawings in which
- Fig. 1: shows schematically a hip implant to be fixed precisely to a femur bone with two carriers with three targets respectively fixed to the implant and femur bone, and
- Fig. 2: shows schematically the configuration of Fig. 1 in combination with a surgeon carrying a movable head-mounted camera, a patient, a computer and a display.

During hip replacement surgery, an implant 1 needs to be placed precisely relative to the undamaged part of the femur bone 2. Due to the nature of the procedure, it is impractical and/or impossible to keep the femur bone 2 still and it is further impractical to maintain a suitable, stable camera position as the camera will get in the way of the surgeon. The present invention provides for a moving or movable camera 3, for example head-mounted worn by the surgeon 4, thus always providing a clear view into the wound area without obstructing the surgeon's view or movement, a carrier 5 with three targets 6 mounted to the undamaged part of the femur bone 2 providing a continuously updated reference locked to the femur bone 2, a carrier 7 with three targets 6 mounted to the implant 1 providing continuous updates on the relative position of the implant 1 relative to the undamaged part of the femur bone 2, a computer 8 and a display 9. The computer 8 receives images from the camera 3 and calculates the relative positions and orientations of the carriers 5 and 7 with three targets 6, respectively - thereby enabling comparison between the desired and current position and orientation of the implant 1 relative to the femur bone 2 as long as the carriers 5 and 7 with targets 6 are within the field of view of the camera 3. This enables the system to compute and display to the surgeon 4 how to move the implant 1 relative to the femur bone 2 in order to achieve the desired position.

The surgeon 4, the patient 10 and the implant 1 may move freely relative to each other during the measurement. The camera 3 captures images of the carriers 5 and 7 with targets 6 essentially simultaneously, or - depending on the required accuracy - closely enough in time to calculate a consistent set of relative positions and orientations for the carriers 5 and 7 with targets 6. The computer 8 provides a display 9, either on the computer 8 itself or remotely located in a suitable location for viewing during the procedure, showing how the implant 1 should be moved relative to the patient 10 to reach the desired relative position.

### Reference numerals:

- 1: implant
- 2: femur bone
- 3: camera
- 4: surgeon
- 5: carrier
- 6: target
- 7: carrier
- 8: computer
- 9: display
- 10: patient

## Claims

1. A system for enabling precision placement of an implant (1) in a patient (10) undergoing surgery, comprising:
a) a movable electro-optical camera (3),
b) a first carrier (5) comprising at least 3 targets (6) to be observed by the camera (3), said carrier (5) to be mounted in a known position and orientation relative to the patient (10),
c) a second carrier (7) comprising at least 3 targets (6) to be observed by the camera (3), said carrier (7) to be mounted in a known position and orientation relative to the implant (1),
d) a computer (8) for calculating the relative positions of the first and second carriers (5, 7) with at least 3 targets (6) based on observations from the movable camera (3).

2. The system according to claim 1, wherein the camera (3) is suited to be moved by a member of the medical staff during measurement.

3. The system according to claim 2, wherein the camera (3) is a head-mounted camera.

4. The system according to any one of claims 1 to 3, wherein the camera (3) captures the image of all targets (6) on the carriers (5, 7) at the same instant.

5. The system according to any one of claims 1 to 4, wherein the computer (8) provides a display (9), either on the computer itself or remotely located in a suitable location for viewing showing how the implant (1) should be moved relative to the patient (10) to reach the desired relative position and orientation.

6. The system according to any one of claims 1 to 5, wherein there are at least three carriers each comprising at least 3 targets to be observed by the camera (3), each carrier to be mounted in a known position and orientation relative to a part of the patient, and implant, or an instrument or any other device whose position is desired to be known.

7. The system according to any one of claims 1 to 6, wherein the camera (3) is a digital camera calibrated to precisely measure spatial directions to targets in space.

8. The system according to any one of claims 1 to 7, wherein the targets (6) are active light emitting elements.
